# EUROPÄISCHE PATENTSCHRIFT

(11) **EP 0 920 641 B1**
(45) Veröffentlichungstag und Bekanntmachung des Hinweises auf die Patenterteilung: **25.10.2000**
(21) Anmeldenummer: 97940070.2
(22) Anmeldetag: 12.08.1997
(51) Int. Cl.: G01S 13/86, G01S 15/02, G01S 13/89

(54) **VERFAHREN ZUR ZERSTÖRUNGSFREIEN DREIDIMENSIONALEN ERFASSUNG VON STRUKTUREN IN BAUWERKEN**
METHOD FOR THREE-DIMENSIONAL AND NON-DESTRUCTIVE DETECTION OF STRUCTURES
TECHNIQUE DE DETECTION TRIDIMENSIONNELLE NON DESTRUCTIVE DES STRUCTURES D'OUVRAGES

(30) Priorität: 22.08.1996 DE 19633813
(43) Veröffentlichungstag der Anmeldung: 09.06.1999
(73) Patentinhaber: Fraunhofer-Gesellschaft zur Förderung der angewandten Forschung e.V., 80636 München (DE)
(72) Erfinder: SCHMITZ, Volker, D-66125 Dudweiler (DE); WIGGENHAUSER, Herbert, D-10555 Berlin (DE); KRAUSE, Martin, D-10965 Berlin (DE); Maierhofer, Christiane, 12107 Berlin (DE)
(74) Vertreter: Schmitt, Meinrad, Dipl.-Ing.
(86) Internationale Anmeldenummer: EP9704371
(87) Internationale Veröffentlichungsnummer: WO9808111

(56) Entgegenhaltungen:
- WO-A-96/19737
- DE-A- 4 239 221
- US-A- 5 005 147
- US-A- 5 384 543

## Beschreibung

Die Erfindung bezieht sich auf ein Verfahren zur zerstörungsfreien dreidimensionalen Erfassung von Strukturen in Bauwerken, insbesondere aus Beton oder ähnlichen Materialien, gemäß den im Oberbegriff des Patentanspruchs 1 angegebenen Merkmalen.

Aus der DE-A-43 20 473 ist ein derartiges Verfahren bekannt, gemäß welchem die Echosignale aus dem Fernfeld eines einzigen Ultraschall-Prüfkopfes dedektierbar sind. Die Amplituden und Laufzeiten der Echosignale sind zusammen mit der Position des Ultraschall-Prüfkopfes in einer Speichereinheit als Laufzeit-Ortskurven mit zugehörigen Amplitudenwerten abspeicherbar. Aus der Gesamtheit der Laufzeit-Ortskurven sind mittels einer Filtervorrichtung, welcher einer bildgebenden Vorrichtung vorgeschaltet sind, auswählbare Anteile der abgespeicherten Ortskurven herausfilterbar.

Bei Ultraschall-Prüfverfahren werden regelmäßig Schallwellen im unteren Frequenzbereich ausgesendet und als Meßgröße wird die Vielfachreflexion von Schallwellen zwischen einem Sende- bzw. Empfangspunkt und dem zu messenden Objekt genutzt. In Frequenzdarstellungen ergeben sich hierbei signifikante Maxima, auf denen bei Kenntnis der Wellengeschwindigkeiten der Abstand zum Objekt berechnet werden kann. In der Druckschrift "Schickert, G (Hrsg.): Vorträge und Plakatberichte Internationales Symposium Zerstörungsfreie Prüfung im Bauwesen, Berichtsband 21, Berlin: DGZfP 1991, 488-504; Wüstenberg H., Möglichkeiten und Konzepte für Ultraschall-Prüfköpfe speziell für das Bauwesen" sind bezüglich des Einsatzes von Ultraschall-Echoverfahren an Bauteilen aus Beton Anregungen zur Anwendung einer künstlichen Apertur gemacht worden. Ziel ist es hierbei, die Schwierigkeiten zu ersetzen, welche bei der Verwendung großflächiger Ultraschall-Köpfe entstehen, durch die Bewegung kleiner vergleichsweise einfach aufgebauter Prüfköpfe und entsprechende Signalverarbeitung. Hierzu wurde an die Unterseite eines Betonblocks ein Beleuchtungsprüfkopf angekoppelt, wobei mittels einer Bohrung ein beugungsbedingter Schatten erzeugt wird. Durch eine numerische Speicherung der mit Amplitude und Phase registrierten Schallsignale und Rekonstruktion mit den Algorithmen der Holographie läßt sich die Größenordnung des Störers ermitteln. Nachteilig ist jedoch, daß infolge einer nicht konstanten Ankopplung merkbare Phasenfehler verursacht werden können, so daß die auf der Grundlage einer phasenempfindlichen Abtastung beruhenden Auswertung unsicher wird.

Ferner gelangen elektromagnetische Radarverfahren zum Einsatz, die auf der Grundlage basieren, daß im zu überprüfenden Material Schichten mit unterschiedlichen dielektrischen Eigenschaften vorhanden sind. Ein derartiges Verfahren ist bekannt aus der Druckschrift "Symposium Zerstörungsfreie Prüfung im Bauwesen, 27.2.-1.3.91 in Berlin, Berichtsband 21, Teil 2, S. 537-544/.; Verfasser: Dipl.-lng. C. Flohrer, Hochtief, Frankfurt/Main; Dipl.-lng. B. Bernhardt, Berlin, Das Orten von Spannbewehrung unter einer mehrlagigen Stahlbetonbewehrung". Mittels einer Antenne werden vorwiegend im Frequenzbereich zwischen 900 MHz und 2 GHz Pulse ausgesandt und empfangen. Die Tiefeninformation wird aus der Laufzeitmessung der reflektierten Signale erhalten, wobei die Ausbreitungsgeschwindigkeit aus der Lichtgeschwindigkeit im Vakuum dividiert durch die Wurzel aus der mittleren Dielektrizitätskonstanten des untersuchten Materials errechnet wird. Typische Werte für Dielektrizitätskonstanten sind beispielsweise für Beton 7, Ziegel 4, Wasser 81 und Eisen unendlich. Da ferner der Wert der Dielektrizitätskonstanten feuchtigkeitsabhängig ist, erfordert die Auswertung einen fachkundigen und erfahrenen Spezialisten. Beim Radarverfahren wird eine Antenne kontinuierlich bewegt und entsprechend gelangt ein Manipulator bei den Ultraschall-Prüfverfahren zum Einsatz, welcher auf dem Prinzip der synthetischen Apertur beruhen. Aufgrund dieser Gemeinsamkeiten kann eine tiefenabhängige Darstellung des untersuchten Objekts durch Aneinanderreihen der aufgenommenen Amplituden ermöglicht werden. Aus den unterschiedlichen Laufzeiten der Signale sowie der Dielektrizitätskonstanten des Materials wird die Tiefenposition der abgebildeten Objekte abgeschätzt, doch ist die Genauigkeit der Ortung in hohem Maß u.a. vom Feuchtigkeitsgehalt des Materials oder von mit Wasser gefüllten Hohlräumen abhängig.

Des weiteren ist aus der Druckschrift "Acoustical Imaging Vol. 19, Edited by Helmut Ermert and Hans-Peter Harjes, Plenum Press, New York and London; Verfasser: Schmitz, V.; Müller, W.; Schäfer G.; Synthetic Aperture Focussing Technique - State of the Art" ein Abbildungsverfahren mit der Bezeichnung: "Synthetic Aperture Focussing Technique - SAFT" bekannt. Dieses Abbildungsverfahren ermöglicht die Berechnung des jeweiligen volumenhaften Bildes eines untersuchten Bereiches.

Schließlich ist aus der SU-A-1 364 868 ein Verfahren zur Messung der Dicke von dielektrischen Gegenständen bekannt. Gemäß diesem Verfahren werden abwechselnd elektromagnetische Wellen und Schallwellen auf den Gegenstand gerichtet und in Abhängigkeit der reflektierten Energie wird die Dicke des Gegenstandes berechnet.

Hiervon ausgehend liegt der Erfindung die Aufgabe zugrunde, das Verfahren der eingangs genannten Art dahingehend weiterzubilden, daß die Genauigkeit der Ortung von Strukturen verbessert wird. Die Ergebnisse sollen von Umgebungsvariablen und Randbedingungen weitgehend unabhängig sein. Des weiteren soll das Verfahren mit einem möglichst geringen Aufwand realisierbar sein.

Das erfindungsgemäße Verfahren zur zerstörungsfreien dreidimensionalen Erfassung von Strukturen gelangt vor allem zur Bauwerksprüfung und Bauwerksüberwachung zum Einsatz. Es ermöglicht eine optimale Bestimmung von Wand- und Fundamentdicken oder von Hohlräumen in Spannkanälen. Des weiteren kann die genaue Lage von Spanngliedern, Spannkanälen und anderen Konstruktionselementen festgestellt werden. Auch kann in zweckmäßiger Weise mit dem erfindungsgemäßen Verfahren eine Ortung und Klassifizierung von Material Inhomogenitäten erfolgen, insbesondere feinen Rissen, welche durch Korrosion von Bewehrungsstäben verursacht werden.

Mit dem erfindungsgemäßen Verfahren wird der zu untersuchende Bereich flächenhaft sowohl mit Radar als auch mit Schall abgetastet, wobei die hochfrequenten, laufzeitabhängigen Daten an jedem Punkt aufgezeichnet werden. Mittels eines Ultraschall-Abbildungsverfahrens, insbesondere der Synthetic Aperture Focussing Technique, nachfolgend SAFT genannt, welches ein dreidimensinales Verfahren ist, wird ein gemeinsamer Volumenbereich bildhaft erzeugt. Es werden wahlweise beliebige Schichtdicken und wahlweise diese Schichten in beliebigen Richtungen dargestellt. Diese Schichten können sowohl parallel zur Meßfläche als auch senkrecht zur Meßfläche vorgegeben werden. In zweckmäßiger Weise wird anhand von "Fingerprints" eine örtliche Kalibrierung der jeweiligen Volumenbereiche durchgeführt. Darüber hinaus wird bei der Volumenrekonstruktion mit dem besonderen und beschriebenen Integralverfahren das Signal-Rausch-Verhältnis verbessert. Die Daten werden in beliebigen Schnittebenen miteinander verknüpft, wobei aufgrund der ortsgenauen Verknüpfung eine quantitative Analyse des Volumenbereiches durchgeführt wird. Somit wird eine Bestimmung unbekannter Materialien über die Kenntnis der Dielektrizitätskonstanten und der Feuchteverteilung ermöglicht.

Die Erfindung wird nachfolgend in der Zeichnung anhand besonderer Ausführungsbeispiele beschrieben, ohne daß die Erfindung hierauf eingeschränkt wird. Es zeigen:
- Fig. 1: ein Flußdiagramm zur Erläuterung der grundlegenden Verfahrensschritte,
- Fig. 2: eine schematische Darstellung zur Erläuterung der Datenerfassung mittels Ultraschall,
- Fig. 3: eine Variante zur Datenerfassung mittels Ultraschall,
- Fig. 4: schematisch die Anordnung eines Prüfkopfes,
- Fig. 5: schematisch die Anordnung zur Datenerfassung mittels Bodenradar,
- Fig. 6: die kombinierte Anordnung von Ultraschall-Prüfkopf und Radarantenne,
- Fig. 7: schematisch die Bilderzeugung.

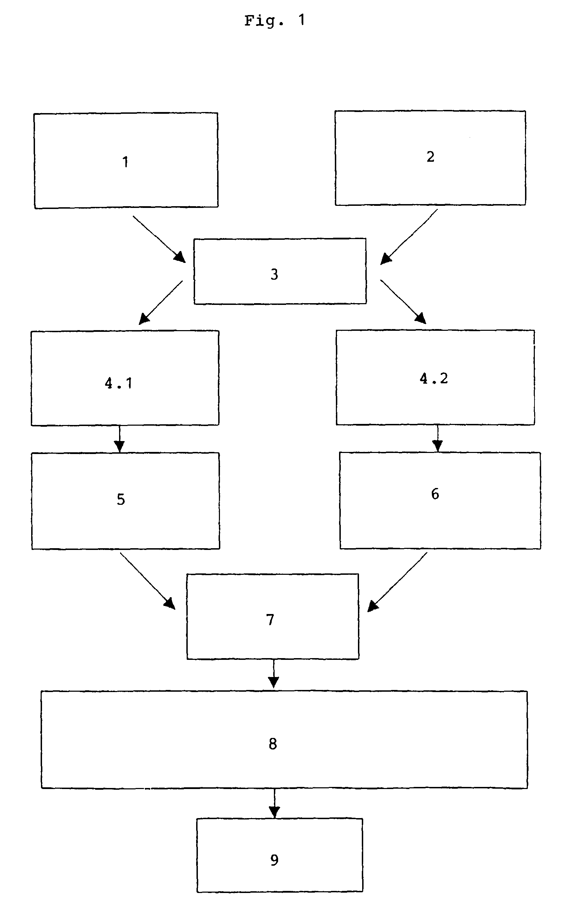

Anhand von Fig. 1 soll der grundsätzliche Verfahrensablauf näher erläutert werden, wobei den einzelnen Funktionsblöcken die nachfolgenden Funktionen zuzuordnen sind. Die Oberfläche des zu untersuchenden Bauteils wird flächenhaft abgetastet und gemäß Block 1 erfolgt zunächst die Abtastung mittels Ultraschall. In einem zweiten Schritt wird der Ultraschall-Sensor ausgewechselt und durch eine Radarantenne gemäß Block 2 ersetzt. Die Radarantenne wird ebenfalls rasterartig bewegt und erfaßt Signale aus dem gleichen Volumenbereich unterhalb der abgetasteten Fläche. Die erfaßten Signale sind pro Ort des jeweiligen Sensors hochfrequente Signale, deren Amplituden von der jeweiligen Bodenstruktur und deren zeitliche Form von dem Abstand der jeweiligen Struktur von der Oberfläche abhängen. Sämtliche in den Verfahrensschritten 1 und 2 erfaßten zeitabhängigen Signale werden in einen Speicher 3 eingegeben und gespeichert.

Aus den gespeicherten Daten wird in Abbildungsverfahren gemäß der Blöcke 4.1 und 4.2, insbesondere gemäß der "Synthetic Aperture Focussing Technique - SAFT", das jeweilige volumenhafte Bild des untersuchten Bereiches ermittelt, wie es nachfolgend noch näher zu erläutern ist. Es sei ausdrücklich festgehalten, daß aus dem untersuchten Volumenbereich keine zweidimensionalen Schnittebenen abgebildet werden, sondern für jede der beiden Verfahrenskomponenten Schall und Bodenradar jeweils ein dreidimensionales Volumen berechnet wird. Hierbei ist von Bedeutung, daß gemäß Block 5 die Strukturdarstellung des untersuchten Bereiches mittels Schall auf der Grundlage von Dichte- und Schallgeschwindigkeitsunterschieden beruht und daß demgemäß Block 6 die Strukturdarstellung des untersuchten Bereiches mittels Bodenradar von der Dielektrizitätskonstanten und folglich auch von der Feuchteverteilung abhängt. In einem erfindungswesentlichen Schritt erfolgt eine tiefenabhängige Kalibrierung der auf den beiden Verfahrenswegen erhaltenen Abbildungen. Gemäß Block 7 erfolgt somit ein Bildvergleich und eine Maßstabseichung anhand örtlicher Fingerprints. Es wurde erkannt, daß bei der dreidimensionalen Abbildung Strukturen vorliegen, welche in den beiden voneinander unabhängigen Abbildungsverfahren identifiziert werden können. Diese mit den beiden Verfahrenskomponenten gemeinsam erfaßten Strukturen, welche als Fingerprints bezeichnet werden, werden in besonders zweckmäßiger Weise zur Anpassung der Maßstäbe der beiden Abbildungen aufeinander erfindungsgemäß genutzt.

Es sei angemerkt, daß bei geologischen Untersuchungen vorwiegend Schnittebenen senkrecht zur Oberfläche abgebildet werden, welche regelmäßig als die y-z-Ebene bezeichnet wird. Durch das erfindungsgemäß vorgeschlagene Verfahren ist es nunmehr möglich, sowohl bei der Datenerfassung mittels Ultraschall als auch mittels Bodenradar Schnittebenen in beliebigen Ebenen auszusuchen und die bildhafte Darstellung der Bodenstrukturen mittels Bildverarbeitung zu verknüpfen, wie es im Funktionsblock 8 angedeutet ist. Bei der Bildverarbeitung können die Abbildungsstrukturen durch Ver-UND-ung, Ver-ODER-ung-, Maximumsbildung oder anderen Methoden optimiert dargestellt werden. In zweckmäßiger Weise richtet sich die Darstellung nach der Bodenstruktur. Ist die horizontale Strukturänderung von Interesse, so wird aus dem rekonstruierten Volumenbereich eine horizontale Schicht in einem vorgebbaren Dickenbereich ausgewählt. Ergeben sich hieraus interessante Schlußfolgerungen für den vertikalen Verlauf, so sind erfindungsgemäß entsprechende vertikale Schichtdickenebenen auswählbar. Darüber hinaus ist es im Rahmen der Erfindung auch möglich, den untersuchten Volumenbereich perspektivisch unter beliebigem Raumwinkel zu betrachten.

Nachdem eine maßstabsgetreue Darstellung gesichert und die Bauteilstruktur optimal dargestellt ist, erfolgt die Analyse gemäß Block 9. Diese beruht auf der Identifikation von Bereichen erhöhter Schallreflektion sowie auf der Identifikation von Bereichen unterschiedlicher Dielektrizitätskonstanten. Durch das Zusammenwirken der beiden physikalischen Parameter wird erfindungsgemäß ermöglicht, beispielsweise über die Maßstabsbeziehung die örtliche Dielektrizitätskonstante und aus dieser die Feuchtigkeit des untersuchten Bodenbereiches zu bestimmen. Ferner können über die Eichung des Bildes, welche auf der Grundlage der Ultraschall-Messung erfolgt, unbekannte Dielektrizitätskonstanten bestimmt werden, um hieraus Rückschlüsse über unbekannte Materialien zu ziehen.

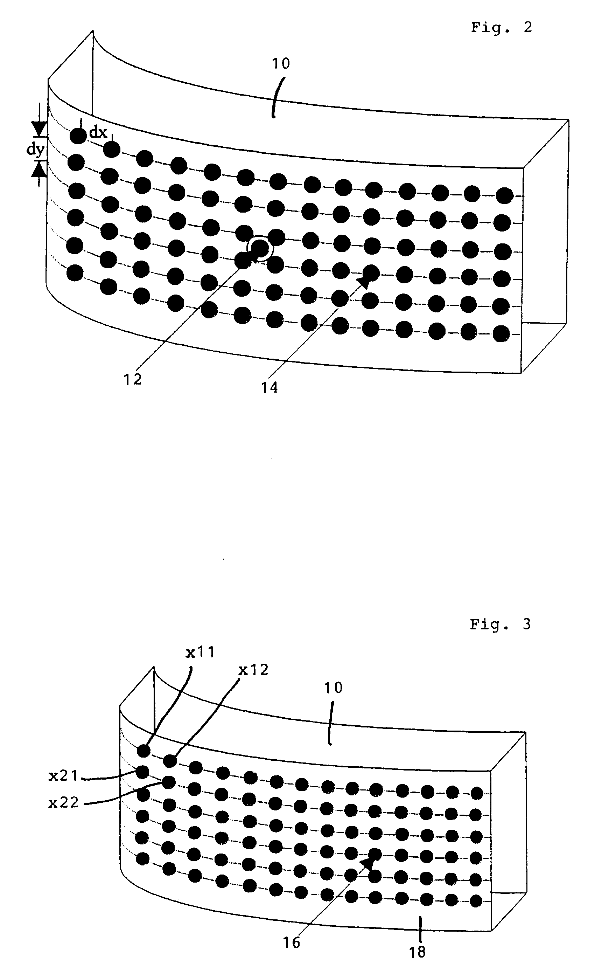

Anhand von Fig. 2 werden die für die Datenerfassung mittels Ultraschall gemäß Funktionsblock 1, in Fig. 1, eingesetzten Prüftechniken erläutert. Bekanntlich können bei der Prüfkopfankopplung auf Strukturen, welche aus Stein oder Beton bestehen, Probleme aufgrund der Oberflächenrauhigkeit des jeweiligen Materials auftreten. Daher ist es nicht unüblich, Mörtel einzusetzen, um einen Prüfkopf ortsfest anzukoppeln. Dies erfolgt mit einem einzigen Prüfkopf 10, welcher als Sender ausgebildet ist. Mittels des Prüfkopfes 10 wird der Schall gesendet, aber nicht empfangen. Der Empfang der reflektierten Signale an den unterschiedlichen Oberflächenpunkten erfolgt durch Abtastung der entsprechenden Positionen mittels eines Laserstrahles. Die Rasterabstände werden den Erfordernissen entsprechend vorgegeben und als typische Rasterabstände haben sich beispielsweise 2 x 2 cm oder 5 x 5 cm als zweckmäßig erwiesen.

In der Ausführungsvariante gemäß Fig. 3 erfolgt die Abtastung mittels eines Ultraschall-Prüfkopfes 12, welcher Schall sowohl aussenden als auch empfangen kann. Dies erfolgt an vorgegebenen Stellen einer ersten Reihe, also an den Stellen x11, x12 ..., einer zweiten Reihe, also x21, x22, ... usw. der Betonwand 10. Um die Probleme der Ankopplung an die Oberfläche 18 zu lösen, gelangt die Methode der Kontakttechnik zum Einsatz.

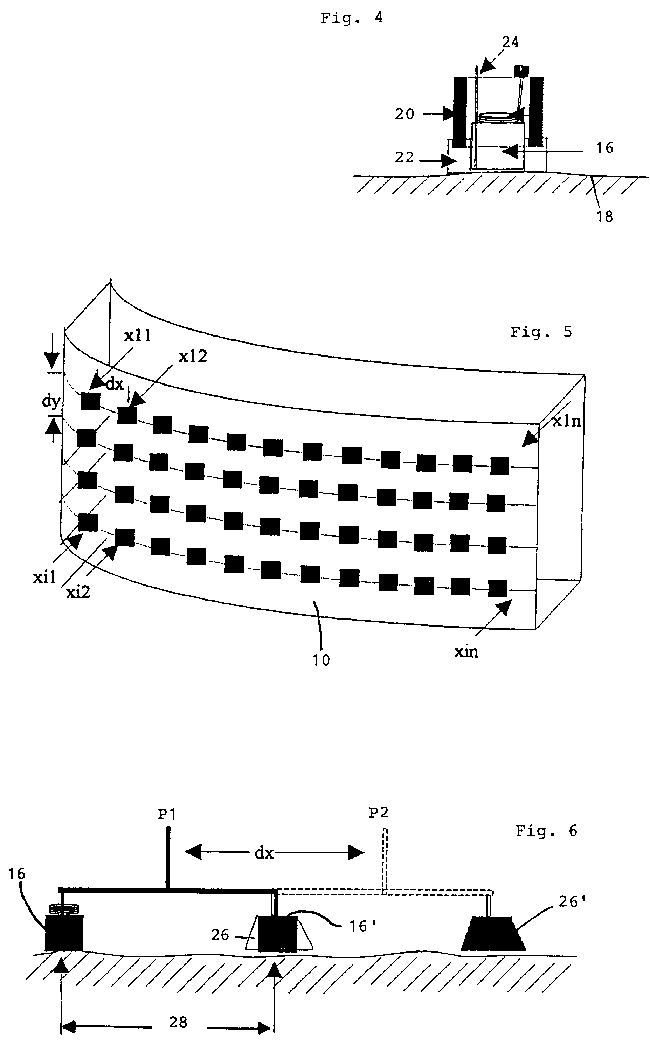

Wie in Fig. 4 dargestellt, ist der kombinierte Prüfkopf 16 in einem Gehäuse 20 angeordnet, welches bezüglich der Betonoberfläche 18 mittels eines Ringes 22, insbesondere aus Gummi oder Styropor kombiniert mit Fett, abgedichtet wird. Durch geeignete Kanäle wird über Schläuche 24 Flüssigkeit, vorzugsweise Wasser, zu- und abgeführt, wobei lediglich das durch die Oberflächenrauhigkeit und die Saugfähigkeit des Bodens verlorene Flüssigkeit/Wasser zu ersetzen ist. Es ist zweckmäßig ein Kreislaufsystem vorgesehen, wobei die Flüssigkeit bzw. das Wasser bei jedem Durchlauf gefiltert wird.

Die Abtastung der Wand mittels Bodenradar soll anhand von Fig. 5 näher erläutert werden. Es erfolgt gleichfalls ein ortsabhängiges Aussenden von Signalen sowie deren Empfang durch Abtastung der Wand bzw. des Bauwerkes. Die Abtastung mittels Bodenradar kann zwar in zweckmäßiger Weise an den gleichen Positionen wie bei der Abtastung mit Ultraschall erfolgen, doch ist dies keine zwingende Voraussetzung. Für unterschiedliche Abtastpositionen wird zusätzlich die relative Lage aller Abtastpunkte zueinander erfaßt und/oder gespeichert.

In einer besonders zweckmäßigen Ausgestaltung gelangt eine Kombisensortechnik gemäß Fig. 6 zur Anwendung, um in vorteilhafter Weise bei der Datenerfassung Zeit einzusparen. Hierbei gelangen die anhand von Fig. 4 und 5 erläuterten Verfahrensschritte zum Einsatz. Erfindungsgemäß wird hierbei der Abstand der Radar- und der Schallsensoren derart vorgegeben, daß er dem Rasterabstand dx multipliziert mit einem ganzzahligen Faktor gleich oder größer 1 entspricht. Hierdurch ist automatisch sichergestellt, daß die Einschallorte von Prüfschuß zu Prüfschuß für die gewählten Sensoren identisch übereinstimmen. In der Position P1 sind der Ultraschall-Prüfkopf 16 und die Radarantenne 26 mit ausgezogenen Linien dargestellt. Der Ultraschall-Prüfkopf 16 und die Radarantenne 26 sind in einem Abstand 28 zueinander angeordnet. In der nächsten Position P2, welche mit gestrichelten Linien angedeutet ist, sind der Ultraschall-Prüfkopf um den Weg dx versetzt. Da die jeweiligen Abstände dx der Meßpunkte entsprechend den Positionen P1 und P2 erfindungsgemäß dem Abstand 28 der Sensoren entsprechen, befindet sich in der Position P2 der Ultraschall-Prüfkopf an der gleichen Stelle wie die Radarantenne in der Position P1.

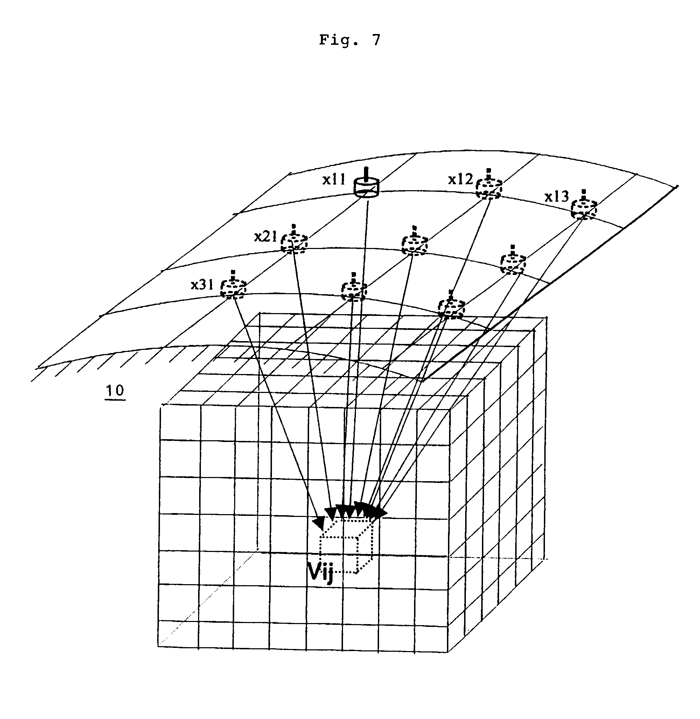

Anhand von Fig. 7 soll die dreidimensionale Bilderzeugung des Abbildungsverfahrens entsprechend den Funktionsblöcken 4.1 und 4.2 von Fig. 1 näher erläutert werden. In zweckmäßiger Weise wird die dreidimensionale Rekonstruktion nach dem Prinzip der dreidimensionalen "synthetischen Apertur" durchgeführt. An den Stellen x11, x12, .. wurden die Meßdaten, vorzugsweise die hochfrequenten Ultraschall- und Radardaten gespeichert. Der zu untersuchende/abzubildende Materialbereich wird in kleine Volumenzellen zerlegt. Typische Größenordnungen liegen im mm- bzw. im cm-Bereich. Die Strecke von den Meßpunkten zu den Volumenzellen wird bereichnet. Mit Hilfe der Lichtgeschwindigkeit und der Dielektrizitätskonstanten ε wird die Strecke für den Bodenradar in Laufzeiten umgerechnet. Ferner wird mit Hilfe der Schallgeschwindigkeit und der Dichte die Strecke für den Bodenschall ebenfalls in Laufzeiten umgerechnet. Somit kann aus den laufzeitmäßig abgelegten Amplitudenwerten eine ortsabhängige Zuordnung in die jeweils betroffenen Volumenzellen erfolgen. Die zu den errechneten Laufzeiten gehörenden Amplituden werden in den Volumenzellen abgelegt bzw. diesen zugeordnet. Die von sämtlichen Meßpunkten aufgenommenen Informationen werden in den Volumenzellen laufzeitkorrigiert aufsummiert. Nachfolgend wird von dem untersuchten Volumenbereich das Videosignal für die Darstellung gebildet. Es erfolgt somit aus den laufzeitmäßig abgelegten Amplitudenwerten eine ortsabhänige Zuordnung in die jeweils betroffenen Volumenzellen. Besonders vorteilhaft ist dieses Verfahren für den bei Beton eingesetzten Frequenzbereich, welcher insbesondere zwischen 50 KHz liegt, wobei die eingesetzten Prüfköpfe Schall in Winkelbereichen vorteilhaft bis zu +/- 90° abstrahlen.

Durch das erfindungsgemäß zum Einsatz gelangende Verfahren SAFT ist es möglich, den Einfluß von Oberflächenwellen und modeumgewandelten Wellen auf die bildhafte Darstellung weitgehend zu unterdrücken. Es ist von besonderer Bedeutung, daß, unabhängig vom Öffnungswinkel der Radarantenne oder vom Schall, die reflektierenden Inhomogenitäten im Boden ortsgetreu zugeordnet werden. In dem letzten Verfahrensschritt wird in dem verarbeiteten Volumenbereich das Videosignal gebildet und das Ergebnis in beliebigen Schichtebenen und Schichtrichtungen dargestellt.

In einer besonderen Ausgestaltung wird bei der Rekonstruktion nach dem Prinzip der synthetischen Apertur eine spezielle Variante der Signalauswertung vorgegeben, nämlich eine der Pulsenergie proportionale Größe. Es sei festgehalten, daß bei den bekannten SAFT-Algorithmen für jedes Volumenelement die Signalintensität zu dem entsprechenden Zeitpunkt bei jeder Prüfkopf-Position aufsummiert wird. Eine Verbesserung der Auflösung und des Signal-Rausch-Abstandes wird in zweckmäßiger Weise jedoch dadurch erreicht, daß eine der Pulsenergie proportionale Größe ausgewertet wird. Erfindungsgemäß wird zu diesem Zweck für jedes Volumenelement das Quadrat der Ultraschall-Intensität gebildet und dieses von dem erwarteten Einsatzpunkt des Echos für die gesamte Pulsdauer integriert. Da die Pulslängen am jeweiligen Ort aufgrund der frequenzabhänigen Schallschwächung und Mikrowellenschwächung nicht exakt bekannt ist, wird in zweckmäßiger Weise in einer Vorabsimulation an einem der im Volumen vorhandenen bekannten Reflektor die Pulsbreite in der Auswertung variiert und für den jeweiligen Tiefenbereich der richtige Wert bestimmt.

### Bezugszeichen

- 1: Datenerfassung mittels Ultraschall
- 2: Datenerfassung mittels Bodenradar
- 3: Speicher
- 4: Abbildungsverfahren
- 5: Strukturdarstellung unter Berücksichtigung von Dichte und Schallgeschwindigkeitsunterschieden
- 6: Strukturdarstellung unter Berücksichtigung von Unterschieden von Dielektrizitätskonstanten
- 7: Bildvergleich
- 8: Bildverarbeitung
- 9: Darstellung
- 10: Betonwand
- 12: Prüfkopf-Sender
- 14: Empfänger
- 16: kombinierter Prüfkopf
- 18: Oberfläche
- 20: Gehäuse
- 22: Ring
- 24: Schlauch
- 26: Radarantenne
- 28: Raster-Abstand

## Patentansprüche

1. Verfahren zur zerstörungsfreien dreidimensionalen Erfassung von Strukturen in Bauwerken, insbesondere aus Beton oder ähnlichen Materialien, gemäß welchem der zu untersuchende Bereich flächenhaft mit Ultraschall abgetastet und die hochfrequenten laufzeitabhängigen Daten für die einzelnen Punkte aufgezeichnet werden und in einem Abbildungsverfahren das jeweilige volumenhafte Bild des untersuchten Bereiches ermittelt wird,
dadurch gekennzeichnet, daß der zu untersuchende Bereich sowohl mit Ultraschall als auch mit Radar abgetastet wird und daß eine Strukturdarstellung des untersuchten Bereiches sowohl auf der Grundlage der mittels Schall erfaßten Daten als auch auf der Grundlage der mittels Radar erfaßten Daten erfolgt und daß diese beiden Darstellungen kalibriert werden.

2. Verfahren nach Anspruch 1, dadurch gekennzeichnet, daß zur vorzugsweise tiefenabhängigen Kalibrierung Strukturen genutzt werden, welche sowohl in der Strukturdarstellung aufgrund der Schall-Datenerfassung als auch in der Strukturdarstellung aufgrund der Radar-Datenerfassung vorhanden sind und/oder daß mittels dieser Strukturen, welche insbesondere als Fingerprints bezeichnet und in den beiden voneinander unabhängigen Verfahrensschritten identifiziert werden, die Anpassung der Maßstäbe der beiden Darstellungen durchgeführt wird und/ oder daß anhand derartiger Strukturen bzw. Fingerprints eine örtliche Kalibrierung der jeweiligen Volumenbereiche durchgeführt wird.

3. Verfahren, insbesondere nach Anspruch 1 oder 2, dadurch gekennzeichnet, daß die Abtastung mittels Schall und die Abtastung mittels Radar an den gleichen Positionen durchgeführt wird oder daß bei Abtastung an unterschiedlichen Positionen die relative Lage aller Abtastpunkte zueinander bekannt und erfaßt ist.

4. Verfahren nach einem der Ansprüche 1 bis 3, dadurch gekennzeichnet, daß als Ultraschall-Abbildungsverfahren die Synthetic Aperture Focussing Technique - SAFT zum Einsatz gelangt, wobei das jeweilige volumenhafte Bild des untersuchten Bereiches berechnet wird und/oder daß ein dreidimensionales Volumen sowohl aus den mittels Ultraschall erfaßten Daten als auch aus den mittels Radar erfaßten Daten bestimmt wird.

5. Verfahren nach Anspruch 4, dadurch gekennzeichnet, daß mittels des dreidimensionalen SAFT-Ultraschall-Abbildungsverfahrens ein gemeinsamer Volumenbereich bildhaft erzeugt wird und/oder daß beliebige Schichtdicken und/oder derartige Schichtdicken in vorgebbaren Richtungen darstellbar sind, wobei vorzugsweise Schichtdicken sowohl parallel zur Meßfläche als auch orthogonal zur Meßfläche darstellbar sind.

6. Verfahren nach einem der Ansprüche 1 bis 5, dadurch gekennzeichnet, daß bei der Volumenrekonstruktion das Signal-Rausch-Verhältnis durch ein Integralverfahren verbessert wird und/oder daß für jedes Volumenelement das Quadrat der Ultraschall-intensität gebildet und dieses von dem erwarteten Einsatzpunkt des Echos für die gesamte Pulsdauer integriert wird.

7. Verfahren nach einem der Ansprüche 1 bis 6, dadurch gekennzeichnet, daß die Daten beliebiger Schnittebenen des zu untersuchenden Bereiches erfaßt und miteinander verknüpft werden und/oder daß aufgrund einer ortsgenauen Verknüpfung eine quantitative Analyse des Volumenbereiches durchgeführt wird.

8. Verfahren nach einem der Ansprüche 1 bis 7, dadurch gekennzeichnet, daß unter Berücksichtigung der Dielektrizitätskonstanten und der Feuchteverteilung unbekannte Materialien bestimmt werden.

9. Verfahren nach einem der Ansprüche 1 bis 8, dadurch gekennzeichnet, daß in einer Vorabsimulation an einem bekannten Reflektor, welcher in dem Volumen vorhanden ist, die Pulsbreite in der Auswertung variiert wird und daß der für den jeweiligen Tiefenbereich zutreffende Wert der Pulslängen bestimmt wird und/oder daß somit eine frequenzabhängige Schallschwächung und/oder Mikrowellenschwächung zumindest näherungsweise kompensiert wird.

10. Verfahren nach einem der Ansprüche 1 bis 9, dadurch gekennzeichnet, daß der Ultraschellsensor, mit welchem die Ultraschall-Abtastung erfolgt, und der Radarsensor, mit welchem die Radar-Abtastung erfolgt, einen Abstand aufweisen, welcher dem Einfachen oder einem Vielfachen des Rasterabstandes der Abtastposition entspricht.

## Claims

1. Method for the non-destructive three-dimensional detection of structures in constructions, in particular constructions made of concrete or similar materials, according to which the region to be inspected is scanned over an area using ultrasound and the high-frequency echo-time-dependent data for the individual points are recorded and in an imaging method the respective volume image of the inspected region is determined, characterized in that the region to be inspected is scanned both using ultrasound and using radar and in that a structural representation of the inspected region takes place both on the basis of the data acquired by means of sound and on the basis of the data acquired by means of radar and in that these two representations are calibrated.

2. Method according to Claim 1, characterized in that, for the preferably depth-dependent calibration, use is made of structures which are present both in the structural representation based on the sound data acquisition and in the structural representation based on the radar data acquisition and/or in that the matching of the scales of the two representations is carried out by means of these structures, which are referred to in particular as fingerprints and are identified in the two mutually independent method steps, and/or in that a local calibration of the respective volume regions is carried out using such structures or fingerprints.

3. Method, in particular according to Claim 1 or 2, characterized in that the scanning by means of ultrasound and the scanning by means of radar is carried out at the same positions or in that, where the scanning takes place at different positions, the relative position of all the scanning points with respect to one another is known and acquired.

4. Method according to one of Claims 1 to 3, characterized in that the ultrasonic imaging method employed is the Synthetic Aperture Focussing Technique - SAFT, in which the respective volume image of the inspected region is calculated and/or in that a three-dimensional volume is determined both from the data acquired by means of ultrasound and from the data acquired by means of radar.

5. Method according to Claim 4, characterized in that a common volume region is imaged by means of the three-dimensional SAFT ultrasonic imaging method and/or in that any desired layer thicknesses and/or such layer thicknesses in presettable directions can be represented, it preferably being possible to represent layer thicknesses both parallel to the measurement area and orthogonal to the measurement area.

6. Method according to one of Claims 1 to 5, characterized in that, in the volume reconstruction, the signal-to-noise ratio is improved by an integral method and/or in that, for each volume element, the square of the ultrasonic intensity is formed and this square is integrated by the expected onset point of the echo for the entire pulse duration.

7. Method according to one of Claims 1 to 6, characterized in that the data of any desired sectional planes of the region to be inspected are acquired and linked to one another and/or in that a quantitative analysis of the volume region is carried out on the basis of a positionally exact linkage.

8. Method according to one of Claims 1 to 7, characterized in that unknown materials are determined by taking account of the dielectric constant and the moisture distribution.

9. Method according to one of Claims 1 to 8, characterized in that, in a pre-simulation using a known reflector which is present in the volume, the pulse width in the evaluation is varied and in that the value of the pulse lengths which applies to the respective depth region is determined and/or in that a frequency-dependent acoustic attenuation and/or microwave attenuation is thus at least approximately compensated.

10. Method according to one of Claims 1 to 9, characterized in that the ultrasonic sensor used to perform the ultrasonic scanning and the radar sensor used to perform the radar scanning are at a spacing which corresponds to a one or more than one times the grid element spacing of the scanning position.

## Revendications

1. Procédé d'analyse tridimensionnelle non destructive de structures dans des ouvrages de construction, en particulier en béton ou d'autres matériaux similaires, selon lequel la zone à étudier est balayée en surface avec des ultrasons et les données haute fréquence fonction du temps de parcours sont relevées pour les différents points et l'image spatiale respective de la zone étudiée est déterminée par un procédé de représentation,
***caractérisé en ce que*** la zone à étudier est balayée aussi bien avec des ultrasons qu'avec un radar et ***en ce qu***'une représentation structurelle de la zone étudiée est effectuée à la fois sur la base des données relevées au moyen des sons que sur la base des données relevées par radar, et ***en ce que*** ces deux représentations sont calibrées.

2. Procédé selon la Revendication 1, ***caractérisé en ce que,*** pour le calibrage de préférence fonction de la profondeur, on utilise des structures qui sont présentes aussi bien dans la représentation structurelle résultant du relevé des données par ultrasons que dans la représentation structurelle résultant du relevé des données par radar et/ou ***en ce*** ***qu***'au moyen de ces structures, lesquelles sont qualifiées en particulier d'empreintes digitales et sont identifiées dans les deux étapes de procédé indépendantes, l'ajustement des échelles des deux représentations est effectué et/ou ***en ce*** ***qu***'à l'aide de telles structures ou empreintes digitales, on procède à un calibrage local des zones spatiales respectives.

3. Procédé, en particulier selon la Revendication 1 ou 2, ***caractérisé en ce que*** le balayage par ultrasons ou le balayage par radar est effectué aux mêmes positions ou *en ce que.* en cas de balayage en des positions différentes, la position relative de tous les points de balayage les uns par rapport aux autres est connue et relevée.

4. Procédé selon l'une quelconque des Revendications 1 à 3, ***caractérisé en ce*** ***qu'***on utilise comme procédé de représentation par ultrasons la technique dite de focalisation à synthèse d'ouverture (Synthetic Aperture Focussing Technique), SAFT, l'image spatiale respective de la zone étudiée étant calculée et/ou ***en ce que*** le volume tridimensionnel est déterminé tant à partir des données relevées par ultrasons qu'à partir des données relevées par radar.

5. Procédé selon la Revendication 4, ***caractérisé en ce qu'***au moyen du procédé de représentation par ultrasons SAFT tridimensionnel, une zone spatiale commune est produite en image et/ou ***en ce que*** des épaisseurs de couche quelconques et/ou de telles épaisseurs de couche dans des directions prédéterminables peuvent être représentées, les épaisseurs de couche pouvant être représentées de préférence aussi bien parallèlement à la surface de mesure que perpendiculairement à la surface de mesure.

6. Procédé selon l'une quelconque des Revendications 1 à 5, ***caractérisé en ce que,*** lors de la reconstruction spatiale, le rapport signal/bruit est amélioré par un procédé intégral et/ou ***en ce que***, pour chaque élément de volume, on calcule le carré de l'intensité des ultrasons et on l'intègre pour toute la durée de l'impulsion à partir du moment d'application escompté de l'écho.

7. Procédé selon l'une quelconque des Revendications 1 à 6, ***caractérisé en ce que*** les données de plans de coupe quelconques de la zone à étudier sont relevées et associées les unes aux autres et/ou ***en ce que*,** sur la base de l'association précise dans l'espace, on procède à une analyse quantitative de la zone spatiale.

8. Procédé selon l'une quelconque des Revendications 1 à 7, ***caractérisé en ce que*** des matériaux inconnus sont déterminés en tenant compte des constantes diélectriques et de la répartition de l'humidité.

9. Procédé selon l'une quelconque des Revendications 1 à 8, ***caractérisé en ce que,*** lors d'une simulation préalable sur un réflecteur connu qui est présent dans le volume, la largeur d'impulsion est soumise à variation lors de l'analyse et ***en ce que*** la valeur correcte des largeurs d'impulsion est déterminée pour la plage de profondeur correspondante et/ou ***en ce*** qu'ainsi, un affaiblissement des sons et/ou un affaiblissement des micro-ondes en fonction de la fréquence est compensé au moins approximativement.

10. Procédé selon l'une quelconque des Revendications 1 à 9, ***caractérisé en ce que*** le capteur à ultrasons avec lequel s'effectue le balayage par ultrasons et le capteur radar avec lequel s'effectue le balayage radar présentent une distance qui correspond au simple ou à un multiple de la distance de la position de balayage.
